# EUROPÄISCHE PATENTSCHRIFT

(11) **EP 2 685 960 B1**
(45) Veröffentlichungstag und Bekanntmachung des Hinweises auf die Patenterteilung: **19.12.2018**
(21) Anmeldenummer: 12709083.5
(22) Anmeldetag: 13.03.2012
(51) Int. Cl.: A61K 9/00, A61K 31/137

(54) **VERWENDUNG EINER SPRÜHFÄHIGEN ZUSAMMENSETZUNG ENTHALTEND AMBROXOL**
USE OF A SPRAYABLE COMPOSITION COMPRISING AMBROXOL
MISE EN OEUVRE D'UNE COMPOSITION PULVÉRISABLE CONTENANT DE L'AMBROXOL

(30) Priorität: 14.03.2011 EP 11158043
(43) Veröffentlichungstag der Anmeldung: 22.01.2014
(73) Patentinhaber: Sanofi-Aventis Deutschland GmbH, 65929 Frankfurt am Main (DE)
(72) Erfinder: SAUERLAND, Sandra, 55216 Ingelheim Am Rhein (DE); BONI, Julia, 55216 Ingelheim Am Rhein (DE); PLOHMANN, Bernd, 55216 Ingelheim Am Rhein (DE)
(74) Vertreter: Weickmann & Weickmann PartmbB
(86) Internationale Anmeldenummer: PCT/EP2012/054406
(87) Internationale Veröffentlichungsnummer: WO 2012/123466

(56) Entgegenhaltungen:
- EP-A2- 1 820 493
- WO-A2-2007/023072
- CN-A- 1 695 601
- US-A1- 2005 075 403

## Beschreibung

Die vorliegende Erfindung betrifft Ambroxol Hydrochlorid zur Behandlung von akuter Pharyngitis.

### Hintergrund der Erfindung

Ambroxol Hydrochlorid (trans-4-[(2-Amino-3,5-dibrombenzyl)amino]cyclohexanol Hydrochloride) wurde ursprünglich als mukoregulatorischer und sekretolytischer Wirkstoff entwickelt, da in pharmakologischen Studien festgestellt wurde, dass es die Sekretbildung der Atemwege fördert und die Ziliaraktivität erhöht. Weiterhin wurde festgestellt, dass es eine lokalanästhetische Wirkung zeigt.

Ambroxol in Form von Lutschtabletten (20 mg Wirkstoff) ist beispielsweise unter dem Markennamen Mucoangin® sowie Lysopadol® zur Schmerzlinderung bei akuten Halsschmerzen, die Kennzeichen einer akuten Pharyngitis sind, zugelassen.

Akute Pharyngitis ist ein entzündliches Syndrom des Pharynx und/oder der Mandeln mit unterschiedlichen Ursachen. Akute Pharyngitis ist durch einen schnellen Ausbruch und einen relativ kurzen Verlauf gekennzeichnet. Üblicherweise handelt es sich um eine gutartige und selbstbegrenzende Erkrankung, die nicht länger als 2 bis 4 Tage anhält. Akute Pharyngitis kann Teil einer generellen Infektion der oberen Atemwege sein oder kann als spezifische lokale Infektion des Pharynx auftreten. Die meisten Fälle werden durch eine Virusinfektion hervorgerufen und treten in Verbindung mit einem viralen Infekt oder einer Erkältung auf. Jedoch können auch eine Vielzahl andere Faktoren Halsschmerzen verursachen, beispielsweise Schadstoffe, Allergien, Traumata oder trockene Luft.

Die Behandlungsmethoden können in symptomatische (Schmerzmittel, Steroide, Lokalanesthetica) und ursächliche (Antibiotika, Virustatika) unterteilt werden. Symtomatische Behandlungen zielen auf eine Reduktion der Schmerzen und des Unwohlseins ab, während ursächliche Behandlungen auf die Heilung der Pharyngitis abzielen oder versuchen Langzeitkomplikationen, wie rheumatisches Fieber, zu verhindern.

Da keine spezifische Behandlung für akute (virale) Pharyngitis existiert, werden diese Infektionen dadurch behandelt, dass man die Symptome kontrolliert bis die Virusinfektion abklingt. Da das Hauptsymtom einer Pharyngitis Halsschmerz ist, ist es für den Patienten hauptsächlich von Bedeutung, diesen zu lindern. Folglich stellen lokal aplizierbare und wirkende Stoffe wie Ambroxol Hydrochlorid das Mittel der Wahl zur Behandlung von Halsschmerzen dar.

Es wurde bereits gezeigt, dass die Behandlung mit Ambroxol Hydrochlorid gut verträglich ist und effektiv die Schmerzen von Patienten mit akuter Pharyngitis lindert. Trotz der aussergewöhnlich guten Verträglichkeit von Ambroxol Lutschtabletten sind in Einzelfällen Unverträgichkeiten und Nebenwirkungen grundsätzlich möglich.

Aufgabe der vorliegenden Erfindung war es alternative Darreichungsformen für Ambroxol Hydrochlorid bereitzustellen, welche die bewährte Wirksamkeit gewährleisten. Die Wirkung sollte dabei nach Möglichkeit bei gleicher oder bevorzugt bei geringerer Dosierung des Wirkstoffs eintreten. Die Wirkung sollte dabei nach Möglichkeit schnell einsetzen, d.h. in einer Zeit von weniger als 30 Minuten. Die Wirkung sollte dabei auch über einen längeren Zeitraum anhalten, d.h. für mehrere Stunden, insbesondere mehr als 2 Stunden.

Die US 2005/075403 offenbart Ambroxol-Sprühlösungen zur Behandlung der Pharyngitis, wobei die verwendeten Lösungen des Anwendungsbeispiels 1% (entsprechend 10 mg/ml) Ambroxol in wässrigen Zubereitungen mit 5% Ethanol enthalten und pro Anwendung 15-50mg Ambroxol dosiert werden. Die EP 1 543 826 lehrt in diesem Zusammenhang, dass hierfür hochkonzentrierter Lösungen von Ambroxol Hydrochlorid (≥ 40 mg/ml), die in Form einer Mundspülung oder eines Sprays verabreicht werden, erforderlich sind. Die hohe Konzentration an Ambroxol Hydrochlorid erfordert jedoch aufgrund der begrenzten Löslichkeit besondere Massnahmen bei der Bereitstellung der Lösungen.

### BESCHREIBUNG

Überraschenderweise wurde nun gefunden, dass sprühfähige Zusammensetzungen von Ambroxol Hydrochlorid enthaltend 1 bis 30 mg/ml Ambroxol Hydrochlorid zur Behandlung von akuter Pharyngitis geeignet sind, sofern diese in einer Dosis von 10 mg lokal appliziert werden. Durch diese Vorgehensweise wird eine hohe Wirksamkeit, ein rasches Einsetzen der Wirkung sowie eine hohe Verträglichkeit gewährleistet.

Folglich betrifft die vorliegende Erfindung Ambroxol Hydrochlorid zur Behandlung von akuter Pharyngitis, wobei Ambroxol Hydrochlorid als sprühfähige Zusammensetzung enthaltend 1 bis 30 mg/ml Ambroxol Hydrochlorid in einer Dosis von 10 mg lokal appliziert wird.

Im Rahmen der vorliegenden Erfindung bezeichnet der Ausdruck "lokal appliziert" das unmittelbare Aufbringen des Wirkstoffs auf die betroffene entzündete Region.

Im Rahmen der vorliegenden Erfindung bezeichnet der Begriff "Dosis" die lokal applizierte Menge an Wirkstoff. Die applizierte Dosis an Ambroxol Hydrochlorid beträgt 10 mg. Üblicherweise wird man Ambroxol Hydrochlorid mit Hilfe eines Sprühapplikators auf die betroffene entzündete Region aufbringen. Das Aufbringen des Wirkstoffs kann somit unmittelbar auf die betroffene entzündete Region erfolgen ohne das Zunge und andere Teile des Mund- und Rachensraums mit dem Wirkstoff in Berührung kommen.

Entsprechend betrifft ein weiterer Gegenstand der vorliegenden Erfindung eine Applikatorvorrichtung zum lokalen Applizieren von 10 mg Ambroxol Hydrochlorid umfassend einen Sprühapplikator und einen Behälter enthaltend eine sprühfähige Zusammensetzung enthaltend 1 bis 30 mg/ml Ambroxol Hydrochlorid.

Üblicherweise wird man den Sprühapplikator so auswählen, dass die Dosis von 10 mg durch einmaliges oder mehrmaliges Betätigen der Sprühvorrichtung appliziert wird.

Als besonders Vorteilhaft hat es sich hierbei erwiesen, den Sprühapplikator so auszuwählen, dass die Dosis von 10 mg durch eine geradzahlige Anzahl von Betätigungen des Sprühapplikators, beispielsweise durch 2 oder 4 Betätigungen, appliziert wird. Dies ermöglicht es die vorgesehene Dosis gleichmässig auf gegenüberliegende Seiten der Oropharynx zu applizieren.

Bevorzugt wird man die beschrieben Applikation des Wirkstoffs im Tagesverlauf 1 bis 10 mal durchführen, besonders bevorzugt bis zu 6 mal. Bevorzugt wird man dabei eine Tagesdosis von Ambroxol Hydrochlorid von 1 bis 200 mg/Tag, besonders bevorzugt von 15 bis 60 mg/Tag, applizieren.

Ein weiterer Gegenstand der vorliegenden Erfindung betrifft die Verwendung von Ambroxol Hydrochlorid zur Behandlung von akuter Pharyngitis, wobei Ambroxol Hydrochlorid als sprühfähige Zusammensetzung enthaltend 1 bis 30 mg/ml Ambroxol Hydrochlorid in einer Dosis von 10 mg lokal appliziert wird.

Ein weiterer Gegenstand der vorliegenden Erfindung betrifft eine Methode zur Behandlung von akuter Pharyngitis, wobei Ambroxol Hydrochlorid als sprühfähige Zusammensetzung enthaltend 1 bis 30 mg/ml Ambroxol Hydrochlorid in einer Dosis von 10 mg lokal appliziert wird.

Die sprühfähigen Zusammensetzungen gemäß der vorliegenden Erfindung enthalten bevorzugt Wasser und Ethanol als Lösungsmittel. Hierbei ist der Anteil an Wasser üblicherweise höher als der an Ethanol. Die sprühfähigen Zusammensetzungen weisen üblicherweise einen Gehalt an Wasser von wenigstens 50 Gew.-%, bevorzugt wenigstens 60 Gew.-%, besonders bevorzugt wenigstens 80 Gew.-% und ganz besonders bevorzugt wenigstens 85 Gew.-% auf, jeweils bezogen auf das Gesamtgewicht der Zusammensetzung.

Weiterhin enthalten die sprühfähigen Zusammensetzungen gemäß der Erfindung bevorzugt wenigstens ein Tensid, insbesondere ein nichtionisches Tensid, wie beispielsweise Polyoxyethylensorbitanmonolaurat (Polysorbat). Die Menge an Tensid liegt dabei üblicherweise im Bereich von < 1 Gew.-%, insbesondere im Bereich von 0,05 bis 0,5 Gew.-%, jeweils bezogen auf das Gesamtgewicht der Zusammensetzung.

Bei den erfindungsgemäß verwendeten sprühfähigen Zusammensetzungen handelt es sich insbesondere um wässrige Zusammensetzung, enthaltend 1 bis 30 mg/ml Ambroxol Hydrochlorid, 50 bis 200 mg/ml Ethanol, 0,5 bis 2 mg/ml eines Tensids sowie weitere Zusatzstoffe ausgewählt unter pH-regulierenden Substanzen, Süssstoffen, Aromastoffen und/oder Konservierungsmitteln.

Ein weiterer Gegenstand der vorliegenden Erfindung betrifft demzufolge eine sprühfähige wässrige Zusammensetzung zur Behandlung von akuter Pharyngitis, enthaltend 1 bis 30 mg/ml Ambroxol Hydrochlorid, 50 bis 200 mg/ml Ethanol, 0,5 bis 2 mg/ml eines Tensids sowie weitere Zusatzstoffe ausgewählt unter pH-regulierenden Substanzen, Süssstoffen, Aromastoffen und/oder Konservierungsmitteln. In einer speziellen Ausführungsform bestehen die erfindungsgemäß verwendeten sprühfähigen Zusammensetzungen aus 1 bis 30 mg/ml Ambroxol Hydrochlorid, 50 bis 200 mg/ml Ethanol, 0,5 bis 2 mg/ml eines Tensids sowie weitere Zusatzstoffe ausgewählt unter pH-regulierenden Substanzen, Süssstoffen, Aromastoffen und/oder Konservierungsmitteln.

Der Begriff "wässrige Zusammensetzung" bedeutet in diesem Zusammenhang, dass die erfindungsgemäßen Zusammensetzung Wasser als Lösungsmittel enthalten.

Die sprühfähigen Zusammensetzungen gemäß der Erfindung enthalten bevorzugt 4 bis 25 mg/ml Ambroxol Hydrochlorid.

Üblicherweise weisen die sprühfähigen Zusammensetzungen gemäß der Erfindung eine Viskosität im Bereich von 1.4 bis 1.6 mPa*s auf.

Üblicherweise weisen die sprühfähigen Zusammensetzungen gemäß der Erfindung einen pH-Wert im Bereich von 5.2 bis 5.8 auf.

Die erfindungsgemäße Applikation der ambroxolhaltigen Zusammensetzungen führt zu einem raschen Einsetzen der schmerzlindernden Wirkung mit anhaltendem Effekt.

Im Folgenden wird die Erfindung anhand nicht einschränkender Beispiele näher erläutert.

### BEISPIELE

### A. Erfindungsgemäße Zusammensetzungen

Je 30 ml einer wässrigen Lösung enthaltend 17,86 mg/ml (Lösung 1), 8,93 mg/ml (Lösung 2) oder 4,46 mg/ml Ambroxol Hydrochlorid (Lösung 3), Ethanol (96 %ig, 84,1 mg/ml), Polysorbat 20 (1 mg/ml), Zitronensäuremonohydrat/Dinatriumhydrogenphosphatdihydrat (2,8 mg/ml), Aromastoffe (1,3 mg/ml), Sucralose (0,8 mg/ml) und Wasser wurden durch Mischen der einzelnen Komponenten bereitgestellt. Die erhaltenen Lösungen wurden in Sprühflaschen aus Braunglas abgefüllt.

### B. Applikation der erfindungsgemäßen Zusammensetzungen

Die erfindungsgemäßen Zusammensetzungen werden jeweils durch 4 Sprühstösse von jeweils 140 µl unmittelbar auf das entzündete Gewebe der Oropharynx appliziert.
Lösung 1: die resultierende Dosis (560 µl) beträgt 10 mg Ambroxol Hydrochlorid Vergleichs-Lösung
2: die resultierende Dosis (560 µl) beträgt 5 mg Ambroxol Hydrochlorid Vergleichs-Lösung
3: die resultierende Dosis (560 µl) beträgt 2,5 mg Ambroxol Hydrochlorid Diese Applikation erfolgt bis zu 6 mal täglich.

## Patentansprüche

1. Ambroxol Hydrochlorid zur Behandlung von akuter Pharyngitis, wobei Ambroxol Hydrochlorid als sprühfähige Zusammensetzung enthaltend 1 bis 30 mg/ml Ambroxol Hydrochlorid in einer Dosis von 10 mg lokal appliziert wird.

2. Ambroxol Hydrochlorid nach Anspruch 1, wobei die sprühfähige Zusammensetzung 4 bis 25 mg/ml Ambroxol Hydrochlorid enthält.

3. Ambroxol Hydrochlorid nach einem der vorhergehenden Ansprüche, wobei die sprühfähige Zusammensetzung Wasser, Ethanol und wenigstens ein Tensid enthält.

## Claims

1. Ambroxol hydrochloride for the treatment of acute pharyngitis, where ambroxol hydrochloride is administered locally as a sprayable composition comprising 1 to 30 mg/ml of ambroxol hydrochloride in a dose of 10 mg.

2. Ambroxol hydrochloride according to Claim 1, where the sprayable composition comprises 4 to 25 mg/ml of ambroxol hydrochloride.

3. Ambroxol hydrochloride according to any of the preceding claims, where the sprayable composition comprises water, ethanol and at least one surfactant.

## Revendications

1. Chlorhydrate d'ambroxol pour le traitement de pharyngite aiguë, le chlorhydrate d'ambroxol étant appliqué localement sous forme de composition pulvérisable contenant 1 à 30 mg/ml de chlorhydrate d'ambroxol dans une dose de 10 mg.

2. Chlorhydrate d'ambroxol selon la revendication 1, dans lequel la composition pulvérisable contient 4 à 25 mg/ml de chlorhydrate d'ambroxol.

3. Chlorhydrate d'ambroxol selon l'une quelconque des revendications précédentes, dans lequel la composition pulvérisable contient de l'eau, de l'éthanol et au moins un agent tensioactif.
